# EUROPEAN PATENT APPLICATION

(11) **EP 4 822 031 A1**
(43) Date of publication of application: **14.10.2026**
(21) Application number: 25168959.2
(22) Date of filing: 07.04.2025
(51) Int. Cl.: C12Q 1/04, G01N 33/543, G01N 33/553, G01N 33/569

(54) **NANOPARTICLES FOR SERS, METHOD OF THEIR MANUFACTURE AND THEIR USE FOR BACTERIAL DETECTION**

(71) Applicant: ECOLE POLYTECHNIQUE FEDERALE DE LAUSANNE (EPFL), 1015 Lausanne (CH)
(72) Inventor: El Chazli, Marwan, 1800 Vevey (CH)
(74) Representative: Cohausz & Florack

(57) **Abstract**

Described herein is method for manufacturing a nanoparticle mixture comprising surface-capped nanoparticles for adhesion to bacteria, the method comprising the steps of providing a nanoparticle starting mixture comprising nanoparticles in a liquid, optionally mixing the nanoparticle starting mixture with a non-ionic surfactant to obtain a nanoparticle intermediate mixture, adding a capping molecule to the nanoparticle starting mixture or the nanoparticle intermediate mixture to obtain a nanoparticle mixture, optionally adjusting the pH value of the nanoparticle mixture and optionally removing the non-ionic surfactant. Also described are surface-capped nanoparticles for adhesion to bacteria obtainable by said method and a method for detecting bacteria applying surface-capped nanoparticles. Further described is the use of surface-capped nanoparticles for detecting bacterial contamination in food and/or feed or for monitoring microbiological safety of production lines for food and/or feed.

## Description

The invention relates to a method for manufacturing a nanoparticle mixture comprising surface-capped nanoparticles for adhesion to bacteria. The invention further relates to surface-capped nanoparticles for adhesion to bacteria themselves, to a method for detecting bacteria applying the surface-capped nanoparticles and to the use of the surface-capped nanoparticles for detecting bacterial contamination in food and/or feed.

### BACKGROUND OF THE INVENTION

Microbiological contamination is a major challenge in the food industry, endangering consumers and resulting in significant financial losses to food companies. In addition, also in pharmaceutical and clinical domains, microbiological contamination may be problematic. In a majority of cases, the microbiological contamination stems from bacteria such as pathogenic strains of Escherichia coli, Salmonella and Listeria. Although microbiological contamination is present in several fields, presently, the food industry is exemplarily described hereinafter.

Food production companies incur massive costs when products are recalled. As part of their efforts to reduce the likelihood of sending contaminated products out of their factories, food manufacturers conduct extensive testing for bacteria of both food products and the production environment.

In a typical production cycle in a food factory, when one batch of a product (e.g., pizzas, grains, dairy, etc.) is completed, food processing equipment such as mixers, containers, driers, etc. is cleaned and sanitized in expectation of a new batch. Before a second batch is started, a swab sample is taken on the freshly cleaned processing equipment following the so-called HACCPs (Hazards Analysis and Critical Control Points) principles, which is a widely-used methodology used in the food industry and endorsed by international regulators. The HACCP hazard analysis is used as a quality tool when it comes to the production and handling of food. This methodology defines Critical Control Points (CCPs), which are specific locations in the overall food production process or in a food production line particularly at risk of (bacterial) contamination, or would be the most likely of producing a positive result if the production line were to be contaminated. Those CCPs are usually the ones that are sampled to prevent, eliminate or reduce a hazard caused by bacterial contamination. CCPs are also usually sampled during the process cleaning validation in a normal quality control routine.

The two main methods used for the detection of bacterial contamination in the food industry are bacterial cell cultures and ATP bioluminescence assays.

Bacterial cell cultures are a highly sensitive method for the detection of bacterial contamination. In a typical food industry quality control routine, operators will take samples from the environment (water pipes, food processing equipment, etc.), the raw materials used for production (dairy, grains, additives, etc.) and the final products. Those samples will be taken to an in-house or affiliated lab to be incubated. Usually, the samples are incubated on different culture media, at different temperatures and over different periods of time to provide ideal incubation conditions for a wide range of bacteria to form colonies. Any bacteria in the samples is subsequently identified based on the characteristics of the formed colonies, by further biochemical tests or by the use of matrix-assisted laser desorption/ionization combined with time of flight analysis (MALDI-TOF). If needed polymerase chain reaction (PCR) can also be applied to screen for specific bacterial strains. Despite being a highly sensitive method for detecting bacterial contamination, bacterial cell cultures are of disadvantage in the food industry as they involve a great deal of manual labour and are therefore costly. Furthermore, the analysis result is usually only available several days after sampling. Batches produced during the period in between sampling and analysis delivery have to be discarded, resulting in a significant loss of products. Additionally, perishable foods usually will already have been shipped to consumers in that period of time and will have to be recalled if a bacterial contamination is determined.

The second method used for the detection of bacterial contamination in the food industry are ATP bioluminescence assays. ATP is an organic compound that is a source of energy in cells, including all bacterial cells. Its presence is a general indicator of bacterial cells. A (swab) sample taken from the food product or environment to be analyzed is introduced into an ATP bioluminescence measurement device. In the device, an enzyme turns ATP bioluminescent, the signal is detected and quantified, giving a rough estimate of the number of bacterial units present in the sample. This method is extremely fast as results are usually available less than 30 minutes after sampling. ATP bioluminescence assays, however, provide a less sensitive method for detecting bacterial contamination. Additionally, one major disadvantage is that as ATP is present in any cellular, bacterial or food source and that ATP bioluminescence assays cannot distinguish between the origin of the ATP. The method is thus not capable of sensitively detecting the presence of specific bacteria and is usually only used for generally measuring the efficiency of cleaning protocols.

Despite these methods being applied for the detection of bacterial contamination, bacterial contaminations are regularly missed and are seen as an ongoing problem in the food industry as well as in pharmaceutical and clinical domains. A major issue preventing more thorough control and oversight of the microbiological safety of food products, pharmaceutical products and production lines and clinical domains is the lack of fast and sensitive tools for the detection and identification of bacteria.

In light of that, it becomes apparent that there is a strong technological need for a bacteria detection method that provides a fast but also specific and sensitive detection of bacterial contamination in an industrial setting.

Thus, it is an object of the present invention to provide means for fast, specific and sensitive detection of bacterial contamination in an industrial food and/or feed and/or pharmaceutical and/or clinical setting and that at least partly overcomes the shortcomings of the approaches currently used for this purpose.

Other and further objects, features and advantages of the present invention will become apparent more fully from the following description.

### SUMMARY OF THE INVENTION

Some or all of these objects are achieved with the present invention by the manufacturing method for surface-capped nanoparticles according to claim 1, the surface-capped nanoparticles according to claims 7 or 9, the method for detecting bacteria according to claim 10, and the use of the surface-capped nanoparticles according to claim 17.

It was surprisingly found that the surface capping of the nanoparticles according to the invention allows the nanoparticles to bind selectively to the surface of bacteria, wherein bacteria/nanoparticle complexes are formed from which Raman spectra can be obtained that allow detection and/or identification of bacteria, in particular in food, feed, pharmaceutical products, and/or clinical settings. This was particularly pronounced for gold nanoparticles capped with amine-group bearing capping molecules that displayed a good adsorption efficiency to gram-negative bacteria.

Especially when using Surface-Enhanced Raman Spectroscopy (SERS), these bacteria/nanoparticle complexes appear to exhibit a selectively enhanced bacterial Raman signal over the background of the analyzed sample, allowing the sensitive detection of bacteria in complex samples by SERS.

It was also surprisingly found that no significant damage to the bacterial membranes is caused by the capped nanoparticles within a timeframe that is pertinent for SERS measurements.

The invention therefore provides a method for fast, specific and sensitive detection of bacterial contamination in an industrial food and/or feed and/or pharmaceutical and/or clinical setting by SERS applying the surface-capped nanoparticles according to the invention.

### DETAILED DESCRIPTION OF THE INVENTION

Further advantageous embodiments according to the invention are specified in the dependent claims and are elucidated in detail herein below.

In a first aspect the invention provides a surface-capped nanoparticle for adhesion to bacteria.

According to an embodiment of the invention, the surface-capped nanoparticles according to the invention are metallic nanoparticles. Preferably the surface-capped nanoparticles according to the invention are noble metal nanoparticles. More preferably the surface-capped nanoparticles according to the invention are gold and/or silver nanoparticles. Most preferred the surface-capped nanoparticles according to the invention are gold nanoparticles. Metallic nanoparticles are elected in the frame of the invention as the best solution for the intended use according to the invention, that is, a method for detecting bacteria by acquiring surface-enhanced Raman spectra (SERS) of bacteria/nanoparticle complexes. Non-metallic nanoparticles decorated or doped with metallic elements such as noble metals, including for instance gold and/or silver, may be envisaged in the context of the invention.

The surface-capped nanoparticles may have different sizes. Advantageously, the surface-capped nanoparticles are nanoscopic and/or can form a colloidal solution or suspension. According to an embodiment of the invention, the surface-capped nanoparticles have an average diameter of from 5 nm to 500 nm determined by transmission electron microscopic methods known to the skilled person. Preferably, the surface-capped nanoparticles have an average diameter of from 10 nm to 300 nm. Most preferred, the surface-capped nanoparticles have an average diameter of from 20 nm to 200 nm. Within this size range the surface-capped nanoparticles are particularly suitable for use in surface-enhanced Raman spectroscopy (SERS) to increase the intensity of the Raman signals of the analyte molecules, allowing their sensitive detection.

Advantageously, the capping molecule of the surface-capped nanoparticles is positively charged or can be easily converted to have positive charges, for example by protonation. Preferably, the capping molecule has the general formula (I) HS-(CR¹R²)ₙ-Y, wherein Y is a functional group with a positive charge or a functional group that can be converted to have a positive charge by protonation, n is an integer from 2 to 15 and R¹ and R² are independently of each other H or C₁₋₃-alkyl. Preferably, Y is an amine group or a quaternary ammonium group. Most preferred, Y is an amine group. Preferably, n is an integer from 2 to 10. More preferably, n is an integer from 2 to 6. Preferably, R¹ and R² are independently of each other H.

In a preferred embodiment of invention, the capping molecule of the surface-capped nanoparticles is selected from the group consisting of cysteamine, 3-aminopropanethiol, 6-aminohexanethiol, trimethyl(10-sulfanyldecyl)azanium and mixtures thereof. Most preferred, the capping molecule of the surface-capped nanoparticles is cysteamine.

Further advantageous embodiments of the surface-capped nanoparticles of the invention are specified in detail herein in the description of the manufacturing method for the surface-capped nanoparticles.

Nanoparticles are most commonly surface-capped (i. e., covered) with a capping molecule such as citrate. In solution, these capping molecules provide efficient stabilization of nanoparticles through electrostatic repulsion. As the capping molecules are usually negatively charged, the surface of surface-capped nanoparticles is usually negatively charged.

Bacterial membranes of gram-negative bacteria are also negatively charged. Thus, available surface-capped nanoparticles and bacteria do not form complexes as their negative surface charges repel each other.

One aspect of the present invention is therefore to provide a method for adjusting the surface capping of nanoparticles to increase their affinity to bacterial membranes.

A surface-capped nanoparticle according to the invention is obtainable by the manufacturing method described herein. In particular, the surface-capped nanoparticles described herein are obtainable by the method for manufacturing a nanoparticle mixture described herein.

According to another aspect of the method of the invention, a method for manufacturing a nanoparticle mixture comprising surface-capped nanoparticles for adhesion to bacteria is provided, the method comprising the steps of
(a) providing a nanoparticle starting mixture comprising nanoparticles in a liquid;
(b) optionally mixing the nanoparticle starting mixture with a non-ionic surfactant to obtain a nanoparticle intermediate mixture;
(c) adding a capping molecule to the nanoparticle starting mixture or the nanoparticle intermediate mixture to obtain a nanoparticle mixture;
(d) optionally adjusting the pH value of the nanoparticle mixture;
(e) optionally removing the non-ionic surfactant.

It was found that it is not possible to obtain a surface-capped nanoparticle according to the invention simply by adding a capping molecule according to the invention to a solution containing nanoparticles having a negatively charged surface-capping as this approach results in complete aggregation of the nanoparticles due to electrostatic adhesion effects between the different capping molecules. The method for manufacturing the surface-capped nanoparticles for adhesion to bacteria according to the invention thus particularly enables switching the negative capping of a surface-capped nanoparticle to a capping with a capping molecule according to the invention while avoiding aggregation.

In a first step of the method for manufacturing a nanoparticle mixture comprising surface-capped nanoparticles for adhesion to bacteria, a nanoparticle starting mixture is provided comprising nanoparticles in a liquid. The nanoparticles in the nanoparticle starting mixture can be commercially available nanoparticles. The nanoparticles in the nanoparticle starting mixture can also be provided through methods known by the skilled person such as the Turkevich method (J. Turkevich et al., "A study of the nucleation and growth processes in the synthesis of colloidal gold", Discuss. Faraday Soc., vol. 11, pp. 55-75, 1951).

In a preferred embodiment of the invention, the nanoparticles in the nanoparticle starting mixture have a negatively charged surface-capping. Advantageously, the capping molecule of the surface-capped nanoparticles in the nanoparticle starting mixture is a molecule having a weak bond to the surface of the nanoparticle, which can thus be displaced by a capping molecule having a stronger bond to the surface of the nanoparticle easily. Preferably, the capping molecule of the surface-capped nanoparticles in the nanoparticle starting comprises a carboxylate group such as a citrate.

In one embodiment of the manufacturing method according to the invention, the surface-capped nanoparticles in the nanoparticle starting mixture comprise a metal. Preferably, the surface-capped nanoparticles in the nanoparticle starting mixture comprise a noble metal.

The surface-capped nanoparticles according to the invention preferably have plasmonic properties and/or are plasmonic nanoparticles. The surface-capped nanoparticles according to the invention are therefore particularly suitable for use in surface-enhanced Raman spectroscopy (SERS) to increase the intensity of the Raman signals of the analyte molecules, allowing their sensitive detection.

Nanoparticles containing gold and/or silver show unique plasmonic properties and are therefore especially suitable to be applied in SERS. Thus, more preferably, the surface-capped nanoparticles in the nanoparticle starting mixture comprise gold and/or silver. Most preferred, the nanoparticles in the nanoparticle starting mixture and/or the nanoparticles according to the invention comprise gold, in particular have a core to which capping molecules are attached, the core preferably comprising gold. SERS effects of gold nanoparticles can be enhanced by tuning the absorption maximum of the gold plasmons towards the red and near-infrared (NIR) range, which can lead to significant signal enhancement in SERS analysis. Additionally, gold is more biocompatible. It has also been reported that the adhesion of gold nanoparticles to bacteria causes less damage to bacterial membranes than the adhesion of silver nanoparticles. This can lead to cleaner Raman spectra as bacteria having ruptured membranes release large quantities of biochemicals causing further peaks to arise in Raman spectra.

Other non-metallic materials such as silica may also be present in the nanoparticles according to the invention.

The shape of the nanoparticles according to the invention is not limited in any way.

In an embodiment of the invention, the nanoparticles applied in a method according to the invention have an average diameter of from 5 nm to 500 nm. The average diameter of the nanoparticles according to the invention is determined by measuring the diameter of the core region ("core diameter") of the nanoparticles using transmission electron microscopic (TEM) methods known to the skilled person. The "core diameter" of a spherical nanoparticle corresponds to the average diameter of the entire particle. The "core diameter" of a non-spherical nanoparticle, such as a nanostar, corresponds to the average diameter of the spherical core of the nanoparticle from which, e.g. in case of a nanostar, branches are grown. Preferably, the nanoparticles have an average diameter of from 10 nm to 300 nm. Most preferred, the nanoparticles have an average diameter of from 20 nm to 200 nm.

In one embodiment of the invention, the nanoparticle starting mixture is optionally mixed with a non-ionic surfactant to obtain a nanoparticle intermediate mixture. This intermediate step serves to further stabilize the nanoparticles while switching the capping molecule. Advantageously, the non-ionic surfactant further prevents aggregation of nanoparticles, in particular during centrifugation. In one embodiment according to the invention, the non-ionic surfactant is a polyalkylene glycol ether. In a particularly preferred embodiment according to the invention, the non-ionic surfactant is a polyoxyethylene sorbitan monolaurate or a polyoxyethylene sorbitan monooleate. The non-ionic surfactant may optionally be removed from the nanoparticle mixture in an additional step. Preferably, the non-ionic surfactant is removed by centrifuging and washing or by dialysis. More preferably, the non-ionic surfactant is removed by dialysis.

In an embodiment of the method for manufacturing a nanoparticle mixture comprising surface-capped nanoparticles for adhesion to bacteria according to the invention, the nanoparticle intermediate mixture is incubated for at least 30 minutes. Preferably, the nanoparticle intermediate mixture is incubated at around 25°C.

In step (c) of the method for manufacturing a nanoparticle mixture comprising surface-capped nanoparticles for adhesion to bacteria, a capping molecule is added to the nanoparticle starting mixture or the nanoparticle intermediate mixture to obtain a nanoparticle mixture.

Advantageously, the capping molecule in step (c) of the method for manufacturing a nanoparticle mixture comprising surface-capped nanoparticles for adhesion to bacteria is positively charged or can be converted to carry a positive charge.

Bacterial membranes of gram-negative bacteria are negatively charged. Preferably, a positively charged capping molecule or a molecule bearing a functional group that can be converted to have a positive charge such as an amine group is used for surface capping the nanoparticles according to the invention as electrostatic interactions of the positively charged surface of the surface-capped nanoparticles allows the nanoparticles to bind selectively to the surface of bacteria to be analyzed. This results in a selectively enhanced bacterial Raman signal over the background of the analyzed sample and enables the sensitive detection of bacteria in complex samples by SERS.

Preferably, the positively charged capping molecule has a strong binding affinity to the surface of the nanoparticles applied in step (a) of the manufacturing method according to the invention. The binding preferably occurs via a functional group present in the capping molecule in addition to the functional group bearing the positive charge or being capable of bearing a positive charge. For example, the binding may occur via a thiol group.

In a particularly preferred embodiment of the invention, the capping molecule has the general formula (I) HS-(CR¹R²)ₙ-Y, wherein Y is a functional group with a positive charge or can be converted to have a positive charge by protonation, n is an integer from 2 to 15 and R¹ and R² are independently of each other H or C₁₋₃-alkyl. C₁₋₃-alkyl means an aliphatic hydrocarbon moiety having 1 to 3 carbon atoms in the chain. Preferably, Y is an amine group or a quaternary ammonium group. Most preferred, Y is an amine group. Preferably, n is an integer from 2 to 10. More preferably, n is an integer from 2 to 6. Preferably, R¹ and R² are independently of each other H.

Capping molecules including a thiol group have a strong binding affinity to gold nanoparticles and are therefore advantageous if the nanoparticles applied in step (a) of the manufacturing method according to the invention are gold nanoparticles. Advantageously, a successful capping of gold nanoparticles with thiolated capping molecules can be detected by means of Raman analysis, as a new, relatively broad, peak appears in the Raman spectrum at around 270-300cm⁻¹. This peak can be attributed to Au-S modes.

In an embodiment according to the invention, the capping molecule of the surface-capped nanoparticles is selected from the group consisting of cysteamine, 3-aminopropanethiol, 6-aminohexanethiol or trimethyl(10-sulfanyldecyl)azanium and mixtures thereof. SERS enhancement is highly dependent on the proximity of the nanoparticles to the analyte. Small capping molecules are thus particularly suitable to be applied in the manufacturing method according to the invention. If nanoparticles being capped with small capping molecules adhere to the surface of bacteria, the core of the nanoparticles is in close proximity to the bacteria leading to high signal enhancement in SERS analysis. Most preferred, the capping molecule of the surface-capped nanoparticles is cysteamine.

In an embodiment of the invention, the pH value of the nanoparticle mixture is optionally adjusted. In a further embodiment of the invention, the pH value is adjusted to pH 2 to 7. Preferably, the pH value is adjusted to pH 3 to 6. Most preferred, the pH value is adjusted to pH 3 to 5.

A particularly preferred embodiment of the method for manufacturing a nanoparticle mixture comprising cysteamine-capped gold nanoparticles for adhesion to bacteria according to the invention is described in the following: in a first step, citrate-capped gold nanoparticles having a negatively charged surface are provided in solution. This nanoparticle starting mixture is incubated with polyoxyethylene (20) sorbitan monolaurate as non-ionic surfactant to obtain a nanoparticle intermediate mixture. Additionally, the pH of the nanoparticle intermediate mixture is adjusted to pH 7. In a further step, cysteamine is added to the nanoparticle intermediate mixture as capping molecule to obtain a nanoparticle mixture comprising positively charged cysteamine-capped gold nanoparticles. Afterwards, the pH of the nanoparticle mixture is adjusted to pH 4. Then, the polyoxyethylene (20) sorbitan monolaurate is removed from the nanoparticle mixture by performing two successive centrifugation and washing steps.

A further aspect of the invention is providing a method for detecting bacteria applying the surface-capped nanoparticles according to the invention.

A method for detecting bacteria according to the invention comprises the steps of
(a) taking a sample of a medium or a surface to be analyzed for the presence of bacteria;
(b) contacting the sample with the nanoparticles described herein to form bacteria/nanoparticle complexes comprising bacteria and the nanoparticles described herein;
(c) optionally immobilizing the bacteria/nanoparticle complexes on an analysis surface;
(d) acquiring surface-enhanced Raman spectra (SERS) of the bacteria/nanoparticle complexes on the analysis surface;
(e) optionally identifying the bacteria applying machine learning techniques.

In a first step of the method for detecting bacteria according to the invention, a sample of a medium or a surface to be analyzed for the presence of bacteria is taken.

In an embodiment of the method for detecting bacteria according to the invention, the sample of a medium or a surface to be analyzed for the presence of bacteria is a sample taken of food or feed products and/or the production environment of food and/or feed. Preferably, the sample is taken from the raw materials used to produce food or feed products and/or of the final food and/or feed products. Particularly preferred, the sample is taken from the surface of production lines for producing food and/or feed such as water pipes or food processing equipment. Most preferred, the sample is taken at CCPs of production lines for producing food and/or feed.

In an embodiment of the method for detecting bacteria according to the invention, the sample of a medium or a surface to be analyzed for the presence of bacteria is a sample taken of pharmaceutical products and/or the production environment of pharmaceutical products. In another embodiment of the method for detecting bacteria according to the invention, the sample of a medium or a surface to be analyzed for the presence of bacteria is a sample taken in a clinical setting.

According to a preferred embodiment of the invention, in step b) of the method for detecting bacteria, the sample is contacted with the surface-capped nanoparticles according to the invention by introducing the sample into a liquid containing the nanoparticles according to the invention to form bacteria/nanoparticle complexes comprising bacteria and the nanoparticles according to the invention. In this step of a method according to the invention, the liquid medium is not particularly limited in any way. The liquid medium can be water, a buffer or a cell culture medium. Preferably, the liquid medium has a pH value of pH 3 to 8, more preferably of pH 4 to 7.

The surface-capped nanoparticles according to the invention are capable to form, in the presence of bacteria in the sample, bacteria/nanoparticle complexes with a broad range of bacteria including various genera, species and strains. Thus, the method for detecting bacteria according to the invention enables a label-free detection of various bacteria.

In one embodiment according to the invention, the method for detecting bacteria is suitable for detecting and identifying gram-negative bacteria. Preferably, the method for detecting bacteria is suitable for detecting and/or identifying gram-positive bacteria and/or gram-negative bacteria, preferably gram-negative bacteria. In particular, the method for detecting bacteria is suitable for detecting and/or identifying bacteria belonging to the genus Pseudomonas, Escherichia, Salmonella and/or Listeria. Preferably, the method for detecting bacteria is suitable for detecting and identifying gram-negative bacteria belonging to the genus Pseudomonas, Escherichia and/or Salmonella. More preferably, the method for detecting bacteria is suitable for detecting and identifying gram-negative bacteria belonging to the genus Salmonella and/or Escherichia. Among the bacteria species, the method is very suitable for detecting Escherichia coli, Pseudomonas protegens, Pseudomonas baetica, Pseudomonas sessiligenes, Pseudomonas ogarae, Pseudomonas chlororaphis. Most preferably, the method for detecting bacteria is suitable for detecting and identifying gram-negative bacteria belonging to the genus Salmonella and/or the species Escherichia coli. **In** one embodiment according to the invention, the method for detecting bacteria is suitable for detecting and/or identifying gram-positive bacteria, in particular gram-positive bacteria belonging to the genus Listeria. The suitability of the method for gram-positive bacteria is believed to rely on the fact that although the membrane composition of gram-positive bacteria is different from gram-negative bacteria, it still comprises a negatively-charged component. In a particularly preferred embodiment according to the invention, the method for detecting bacteria is suitable for detecting and/or identifying pathogenic bacteria.

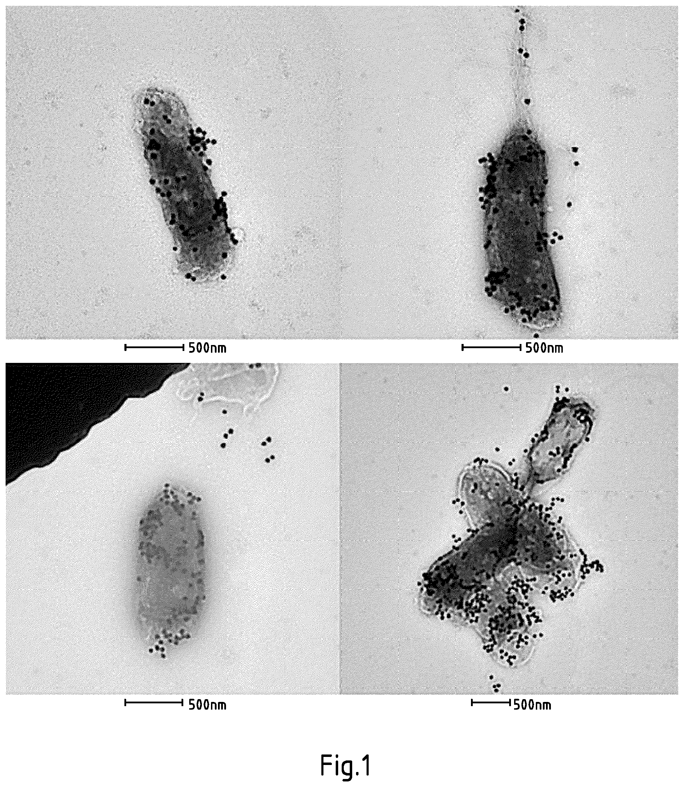

The formation of bacteria/nanoparticle complexes can be confirmed by TEM analysis. Figure 1 shows TEM images of bacteria/nanoparticle complexes acquired after incubation of bacteria and cysteamine-capped gold nanoparticles.

Surprisingly, it was found that cysteamine-capped gold nanoparticles have a remarkable adsorption efficiency to gram-negative bacteria, producing a regular and plentiful coating of the bacteria (cf. Fig. 1). Additionally, it was found that an hour after introducing a sample containing gram-negative bacteria into a liquid containing cysteamine-capped gold nanoparticles according to the invention, the bacterial membranes maintain their integrity. Thus, no significant damage to the bacterial membranes is caused by the cysteamine-capped gold nanoparticles within a timeframe that is pertinent for SERS measurements. Cysteamine-capped gold nanoparticles are therefore preferably applied in a method for detecting bacteria according to the invention.

With the method for detecting bacteria according to the invention, bacteria are advantageously directly detected in a liquid when the bacteria concentration in step (b) is greater than or equal to 10⁶ CFU/mL.

To allow for the detection of bacteria at even lower concentrations, the bacteria/nanoparticle complexes may be immobilized on an analysis surface in a further step of a method for detecting bacteria according to the invention. By immobilizing the bacteria/nanoparticle complexes on an analysis surface, the bacteria/nanoparticle complexes are concentrated in a known location and can therefore be detected with greater sensitivity. Hence, in a preferred embodiment according to the invention, the method is suitable for detecting and identifying bacteria at concentrations in step (b) of the method for detecting bacteria of as low as 10² CFU/mL.

In one embodiment of the invention, the bacteria/nanoparticle complexes are immobilized by drying an aliquot of the liquid containing the bacteria/nanoparticle complexes on a slide.

In a particularly preferred embodiment of the invention, the liquid containing the bacteria/nanoparticle complexes does not need to be evaporated to immobilize the bacteria/nanoparticle complexes. A concentration of the bacteria/nanoparticle complexes is achieved in liquid by immobilizing the bacteria/nanoparticle complexes on an analysis surface, i.e. in a smaller volume at a known location. The immobilization of the bacteria/nanoparticle complexes on an analysis surface in a liquid can be achieved for example by using bacteria traps applying optofluidics and optical tweezers. Preferably, the bacteria/nanoparticle complexes are immobilized on an analysis surface in a liquid using microwells attracting bacteria to an analysis surface by electrostatic effects.

In a further step of the method for detecting bacteria according to the invention, surface-enhanced Raman spectra (SERS) of the bacteria/nanoparticle complexes on the analysis surface are acquired.

Raman spectroscopy is a non-destructive analytical technique generally known to the skilled person. It is based on the inelastic scattering of monochromatic light as it interacts with molecular vibrations, rotations, and other low-frequency modes in the analyzed material. When light is scattered by a molecule, most photons are elastically scattered (Rayleigh scattering), but a small fraction undergoes inelastic scattering, resulting in a shift in energy that corresponds to the vibrational modes of the molecule. This shift provides a "chemical fingerprint" which is unique to the analyzed molecule allowing its identification even in complex mixtures.

SERS is an advanced variation of conventional Raman spectroscopy, wherein the signal intensity of the analyte is increased by the proximity of the molecule to be analyzed to plasmonic nanostructures (Pilot et al. "A review on surface-enhanced raman scattering," Biosensors, vol. 9, no. 2, p. 57, 2019). Plasmonic nanostructures are metallic structures in the ranges of a few nanometers to a several hundred nanometers which exhibit unique optical properties due to their small sizes, thanks to the increase in local electric field density induced by the oscillating free electrons of the metal. In case of Raman spectroscopy, as the absorption process leading to scattering is proportional to the local electric field intensity, which is amplified by plasmonic nanostructures. Compared to conventional Raman spectroscopy, the signal in SERS can be increased by up to factor 10¹⁰, allowing the detection of trace levels of target molecules.

The surface-capped nanoparticles according to the invention in particular show plasmonic nanostructures increasing the intensity of Raman signals of bacteria in SERS analysis, allowing their sensitive detection.

In a particularly preferred embodiment of the invention, the SERS spectra are collected from liquid samples.

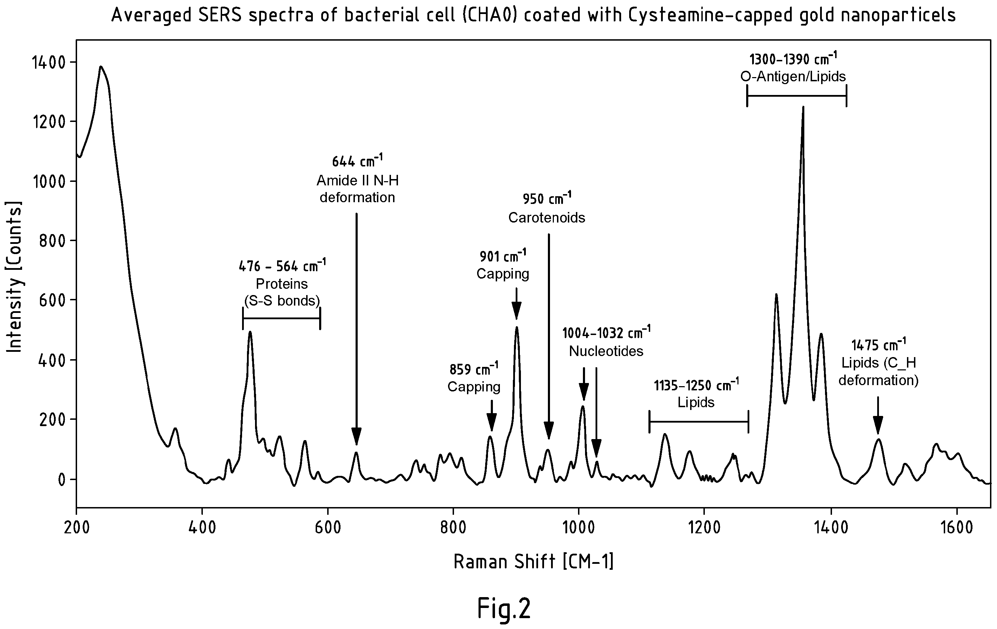

Different genera, species and strains of bacteria show uniquely distributed signals when analyzed using Raman spectroscopy. These characteristic signals may also be referred to as "fingerprints". Acquiring distinct Raman spectra of bacteria can thus be applied for bacteria identification at the species and subspecies level. In a particularly preferred embodiment, the method according to the invention is suitable for detecting and identifying a single bacterial cell. A characteristic annotated averaged SERS spectrum of a single Pseudomonas protegens (CHAO) bacterium coated with cysteamine-capped gold nanoparticles is shown in Figure 2.

Classification of bacteria can be done at different levels of taxonomy. The "fingerprints" of bacteria in the Raman spectra become increasingly similar from genus to species level and especially from species to strain level making the identification of bacteria increasingly difficult.

Therefore, in one embodiment of the invention, the method for detecting bacteria comprises the step of identifying the bacteria applying machine learning techniques.

Machine learning techniques can be advantageously applied for interpreting bacterial Raman spectra and identifying bacteria as subtle differences between the signals in the Raman spectra of closely related bacteria strains are detected.

Suitable machine learning techniques for interpreting bacterial Raman spectra and identifying bacteria are for example random forest classifiers, convolutional neural networks (CNN), support vector machines, and deep learning (DL) techniques. In a particularly preferred embodiment of the invention, identifying the bacteria in step (e) of the method for detecting bacteria according to the invention comprises subjecting the SERS spectra to a Principal Component Analysis (PCA) algorithm. PCA is a statistical technique that reduces the dimensionality of data by highlighting the most significant features.

Preferably, bacteria are identified by employing the following approach: initially, a comprehensive database of bacteria is established by recording reference SERS spectra of various bacteria under standardized conditions. Subsequently, PCA is applied to the recorded SERS spectra transforming the original spectral data into principal components for each bacterium in the database. For bacteria identification, SERS spectra acquired of unidentified bacteria/nanoparticle complexes applying the method for detecting bacteria according to the invention are subjected to the same PCA transformation as the reference spectra. This step involves projecting the unknown spectra onto the principal components derived from the database, resulting in a set of scores that represent its position in the reduced-dimensional space. The final identification step involves comparing the PCA scores of the unknown bacteria to those of the bacteria included in the database using machine learning techniques. By evaluating the PCA scores, the unknown bacteria can be identified as the bacteria in the database whose principal components, i.e., spectral properties show the best match.

Preferably, bacteria are identified applying the method for detecting bacteria according to the invention with a PCA score match of 80% or more. More preferably, bacteria are identified applying the method for detecting bacteria according to the invention with a PCA score match of 85% or more. Most preferably, bacteria are identified applying the method for detecting bacteria according to the invention with a PCA score match of 90% or more.

In a particularly preferred embodiment according to the invention, the method for detecting bacteria is suitable for detecting and identifying bacteria in 1 h or less. Preferably, the method for detecting bacteria is suitable for detecting and identifying bacteria in 30 min or less.

Advantageously, applying the method for detecting bacteria according to the invention bacteria are detected and identified within a few minutes after sampling in an industrial food and/or feed and/or pharmaceutical and/or clinical setting.

In a preferred embodiment according to the invention, the method for detecting bacteria is performed in close proximity to or directly at production lines for food and/or feed and/or pharmaceutical products.

Due to the rapid availability of the analysis results applying the method for detecting bacteria according to the invention, the downtime of production lines during process cleaning validation or a normal quality control routine is reduced. Advantageously, the quantity of products produced between sampling and obtaining the analysis results, which would have to be discarded if a bacterial contamination was determined, is reduced using the method for detecting bacteria according to the invention. More preferred, food and/or feed and/or pharmaceutical products have not been shipped to consumers before the analysis result is available and will not have to be recalled if a bacterial contamination is determined using the method for detecting bacteria according to the invention.

In one embodiment according to the invention, the method for detecting bacteria is suitable for detecting bacterial contamination in food and/or feed. Advantageously, the method for detecting bacteria is also suitable for monitoring bacterial contamination on the surface of containers of food and/or feed products.

In one embodiment according to the invention, the method for detecting bacteria is suitable for detecting bacterial contamination in pharmaceutical products.

In one embodiment according to the invention, the method for detecting bacteria is also suitable for monitoring bacterial contamination on surfaces in clinical settings.

In a preferred embodiment according to the invention, the method for detecting bacteria is suitable for monitoring microbiological safety of production lines for food and/or feed and/or pharmaceutical products. Preferably, the method for detecting bacteria is suitable for monitoring bacterial contamination on the surface of production lines for producing food and/or feed such as water pipes or food processing equipment.

More preferably, the method for detecting bacteria is suitable for confirming cleaning and disinfection protocols of production lines for producing food and/or feed. Most preferable, the method for detecting bacteria is suitable for providing a detailed analysis of bacterial contamination at the CCPs of a production line for producing food and/or feed.

In a further aspect the invention provides a use of the nanoparticles according to the invention for detecting bacterial contamination in food and/or feed and/or pharmaceutical products and/or clinical settings. In particular, the nanoparticles according to the invention can be used for detecting bacterial contamination in food and/or feed and/or pharmaceutical products and/or clinical settings by SERS.

In yet another aspect the invention provides a use of the nanoparticles according to the invention for monitoring microbiological safety of production lines for food and/or feed and/or pharmaceutical products. In particular, the nanoparticles according to the invention can be used for monitoring microbiological safety of production lines for food and/or feed and/or pharmaceutical products by SERS.

### DESCRIPTION OF FIGURES

In the drawings,
Figure 1 shows TEM images of bacteria/nanoparticle complexes acquired after incubation of bacteria and cysteamine-capped gold nanoparticles;
Figure 2 shows an annotated averaged SERS spectrum of a single Pseudomonas protegens (CHAO) bacterium coated with cysteamine-capped gold nanoparticles.

Figure 1 shows TEM images of bacteria/nanoparticle complexes acquired after incubation of bacteria and cysteamine-capped gold nanoparticles. The gold nanoparticles show an efficient coating of the bacteria. Additionally, Fig. 1 shows that the bacterial membranes maintain their integrity and that the cysteamine-capped gold nanoparticles do not cause significant damage to the bacterial membranes.

Figure 2 shows an annotated averaged SERS spectrum of a single Pseudomonas protegens (CHAO) bacterium coated with cysteamine-capped gold nanoparticles. The spectrum is dominated by signals belonging to lipid membranes (Lipopolysaccharides and O-Antigen), with notable peaks at 1091-1099cm⁻¹ and 1115-1125 cm⁻¹ attributed to C-C vibrations. The signals at 1495 cm⁻¹ are linked to glycosidic linkages. The signals at 1576 cm⁻¹ are linked to the N-H bend of N-acetylglucosamine. Signals originating from proteins and nucleotides are also present, including two nucleotide peaks at 1004 and 1032 cm⁻¹ and several protein related signals between 475 and 564 cm⁻¹. A few signals caused by the capping molecule cysteamine are also present, including signals at 859 and 901 cm⁻¹. The spectrum was recorded applying a laser power of 1.1 mW, the objective was 100X and 0.75 NA and the integration time 500ms.

In the following, the invention is further described by way of examples that are in no way meant to be limiting.

### EXAMPLES

### MATERIALS AND ANALYTICAL METHODS

Deionized (DI) water and MilliQ water were used for all buffers, solutions and dilutions. (4-(2-hydroxyethyl)-1-piperazineethanesulfonic acid) (HEPES) dissolved in MilliQ water at 1 M was used for the synthesis of gold nanostars. Cysteamine (>98%) in powder form from Sigma-Aldrich was used and diluted in water. Tween 20 and Tween 80 from ThermoFisher Scientific were used and diluted with DI Water. Sodium citrate tribasic dihydrate was diluted in DI water as per needed for nanoparticle synthesis and preparation of citrate buffers. A variety of buffers was prepared with analytical grade chemicals, including borate buffer (boric acid + sodium tetraborate), citrate buffer (sodium citrate + citric acid) and acetate buffer (sodium acetate + acetic acid).

All Raman spectra were acquired using a Renishaw inVia Raman Microscope, either applying a 532 nm or a 785 nm laser.

SERS spectra of single bacterial cells were acquired using the Renishaw inVia Raman Microscope applying a laser at 785 nm by focusing on an individual cell visibly coated with nanoparticles. Although single nanoparticles are not visible on an optical microscope, aggregates can be large enough to become visible. In this case, the area of the whole cell was scanned with a 100x 0.75 NA objective and all recorded spectra were averaged.

### SYNTHESIS OF NANOPARTICLES

### a) SYNTHESIS OF GOLD NANOSTARS USING HEPES BUFFER

Four batches of gold nanostars were synthesized with HEPES buffer by the following procedure: a 1 M solution of HEPES buffer in MilliQ water at pH 7.4 was diluted at different ratios with DI Water to a total final volume of 30 mL. The final concentration of HEPES was 40, 20, 10 and 5 mM in the four batches, respectively. In each case, the mixture was briefly mixed with a magnetic stirrer to ensure homogeneity, after which 600 µL of 10 mM HAuCl₄ solution was added. The solution was stirred mildly for 1.5 minutes and was then left to react undisturbed for 40 minutes. In that timespan, the solution went from transparent to a dark color, followed by a bluish tint for the two batches with the lowest concentrations of HEPES (10 mM and 5 mM). Prior to use, the solutions of nanoparticles were centrifuged and washed with MilliQ water. Changing the final concentration of HEPES in the solution results in different branch lengths of the gold nanostars. The higher the concentration of HEPES, the more well-defined and longer the branching of the nanostars, and the higher the red-shift of the absorption maxima.

The synthesized nanostars can be used to obtain surface-capped nanoparticles according to the invention by the manufacturing method disclosed herein. After functionalization, Raman spectra of surface-capped nanoparticles according to the invention show no HEPES-related peaks indicating a complete replacement of HEPES as the capping molecule.

### b) SYNTHESIS OF CITRATE-CAPPED GOLD NANOPARTICLES

A batch of citrate-capped gold nanoparticles was synthesized using a slightly modified version of the Turkevich method. A solution of 0.25mM HAuCl₄ was prepared in 50 mL of MilliQ water. The pH value was adjusted to pH 6.5 using a concentrated NaOH solution and the mixture was brought close to a boil but not yet fully boiling. Under vigorous stirring, 368 µL of a 34 mM citrate solution (prepared in MilliQ water) were added. Within a few minutes, the solution turned dark and then reddish. The reaction was considered to be finished when the color did not change anymore, and the mixture was left to cool down to room temperature and then centrifuged at 2000 rpm for 10 minutes to remove any excessively large aggregates.

### c) SYNTHESIS OF CYSTEAMINE-CAPPED GOLD NANOPARTICLES BASED ON COMMERCIALLY AVAILABLE GOLD NANOPARTICLES

A batch of cysteamine-capped gold nanoparticles was synthesized by washing a solution of commercially available citrate-capped gold nanoparticles with borate buffer (pH 6.5, 2 mM). Afterwards, a final concentration of 0.1% of Tween 20 was added and the mixture was left to incubate for at least 30 minutes. Then, cysteamine was added to a final concentration of at least 100 µM and the resulting mixture was again incubated for at least 30 minutes. Afterwards, the pH value was lowered by adding a final concentration of sodium acetate buffer pH 4 of 10 mM. Lastly, the mixture was centrifuged and washed twice with a 10 mM sodium acetate buffer pH 4.

A successful capping of the gold nanoparticles with cysteamine was confirmed by Raman analysis, as a new, relatively broad, peak appeared in the Raman spectra at around 270-300cm⁻¹. This peak can be attributed to Au-S modes.

### CHARACTERIZATION AND SURFACE-CHARGE DETERMINATION OF SURFACE-CAPPED NANOPARTICLES

The synthesized nanoparticles were characterized with a UV/Vis spectrophotometer to determine their absorption maximum λₘₐₓ. All synthesized nanoparticles showed an absorption maximum λₘₐₓ of between 744 and 532 nm.

The surface charge of the nanoparticles was qualitatively determined by tuning the pH value of the solution and observing aggregation (or lack thereof). Aggregation at high pH values indicates a positive capping, as the protonated molecules keeping nanoparticles electrostatically stable due to their positive charges become deprotonated at high pH values and vice-versa. Aggregation at low pH values indicates negative capping.

### CHARACTERIZATION OF BACTERIA/NANOPARTICLE COMPLEXES

Standard Transmission Electron Microscopy (TEM) or cryo-TEM were used to image the coating of single bacterial cells with nanoparticles. To prepare for TEM imaging, 50 µL of the nanoparticle solutions at a concentration of at least 10¹¹ were incubated for 30 minutes with 30 µL of Pseudomonas protegens CHAO bacterial solution of a concentration of 10¹⁰ CFU/ml.

### IMAGING AND IDENTIFICATION OF BACTERIA

### a) BACTERIAL STRAINS

A panel of different bacteria was exemplarily used to show the possibility of identifying bacteria by Raman spectroscopy. It was assessed whether the Raman spectra of the selected bacteria shown in Table 1 below correlate with the genetic diversity between the bacterial strains selected. The selection included bacteria belonging to different genera (Escherichia vs. Pseudomonas) and different bacterial species (protegens, baetica, ogarae, chlororaphis, sessilinigenes).

**Table 1: Tested bacterial strains.**

| Genus | Species | Strain |
|---|---|---|
| Pseudomonas | protegens | CHA0 |
| Pseudomonas | protegens | Pf5 |
| Pseudomonas | baetica | a390 |
| Pseudomonas | sessiligenes | CMR12a |
| Pseudomonas | ogarae | F113 |
| Pseudomonas | chlororaphis | PCL1391 |
| Escherichia | coli | DH5α |

### b) BACTERIA PREPARATION PROTOCOLS

All bacteria samples used were taken out of -80°C deep freezer from a glycerol stock containing a nutrient broth mixed with 40% final glycerol as a cryoprotector. The bacterial strains were plated on nutrient agar plate and incubated overnight at 25°C. Bacterial suspensions were prepared by resuspending bacterial colonies into an 1.5 mL Eppendorf tube containing 1mL of sterile distilled water.

### c) IMAGING AND CLASSIFYING BACTERIA IN A SUSPENSION AT 532 NM

A comprehensive database of bacteria was established by recording reference Raman spectra in a range of 500 cm⁻¹ and 1800 cm⁻¹ of the bacteria listed in Table 1 under standardized conditions. Bacteria were prepared fresh from culture in an Eppendorf tube filled with 1 mL of DI water and adjusted to a concentration of 10⁹ CFU/mL. Each solution was briefly vortexed to resuspend the bacteria uniformly and 350 µL were pipetted into a well of a sterile 96-well microplate. Spectra were collected by imaging directly into the liquid, around half a millimeter or less under the surface of the liquid. All spectra were acquired with the Renishaw inVia Raman Microscope, using a 532 nm laser with a power of 40 mW at the sample and a 50x long working distance objective.

The integration time was 30 seconds. Three spectra were collected for each bacterial strain.

### d) PRINCIPAL COMPONENTS ANALYSIS (PCA)

Python scripts were employed to automate the data analysis process. All Raman spectra were pre-processed using the Rampy package, an open-source Raman library written in Python. Pre-processing was done by removing the baseline using an asymmetrically re-weighted penalized least squares algorithm (arPLS) and normalizing using the Standard Normal Variate (SNV) method. Partially, a Whittaker smoothing algorithm was applied to reduce high frequency noises in the spectra, rendering the spectra visually clearer. The reference spectra, recorded between 500 cm⁻¹ and 1800 cm⁻¹, were then run through a PCA algorithm.

For identification of unknown bacteria, SERS spectra acquired of unidentified bacteria/nanoparticle complexes applying the method for detecting bacteria according to the invention were also subjected to the same pre-processing and PCA transformation as the reference spectra. This step involved projecting the unknown spectra onto the principal components derived from the database, resulting in a set of scores. The final identification step involved comparing the PCA scores of the unknown bacteria to those of the bacteria included in the database, wherein three principal components were taken into account. In this way, the possibility of classifying bacteria of the genera Pseudomonas and Escherichia using PCA based on their SERS spectra was established.

## Claims

1. A method for manufacturing a nanoparticle mixture comprising surface-capped nanoparticles for adhesion to bacteria, the method comprising the steps of
(a) providing a nanoparticle starting mixture comprising nanoparticles in a liquid;
(b) optionally mixing the nanoparticle starting mixture with a non-ionic surfactant to obtain a nanoparticle intermediate mixture;
(c) adding a capping molecule to the nanoparticle starting mixture or the nanoparticle intermediate mixture to obtain a nanoparticle mixture;
(d) optionally adjusting the pH value of the nanoparticle mixture;
(e) optionally removing the non-ionic surfactant.

2. The method according to claim 1, **characterized in that** the nanoparticles in the nanoparticle starting mixture have a negatively charged surface-capping, and/or **in that** the nanoparticles comprise a metal, preferably a noble metal, more preferably gold and/or silver, most preferred gold, and/or **in that** the nanoparticles have an average diameter of from 5 nm to 500 nm, preferably from 10 nm to 300 nm, most preferred from 20 nm to 200 nm determined by transmission electron microscopy (TEM), and/or **in that** the non-ionic surfactant in step (b) is a polyalkylene glycol ether, and/or **in that** the nanoparticle intermediate mixture is incubated for at least 30 minutes, preferably at 25°C, and/or **in that** the capping molecule in step (c) is positively charged.

3. The method according to any one of the preceding claims, **characterized in that** the capping molecule has the general formula (I) HS-(CR¹R²)ₙ-Y, wherein Y is a functional group with a positive charge or a functional group that can be converted to have a positive charge by protonation, n is an integer from 2 to 15, preferably from 2 to 10, more preferably from 2 to 6, R¹ and R² are independently of each other H or C₁₋₃-alkyl, preferably H.

4. The method according to claim 3, **characterized in that** Y is an amine group or a quaternary ammonium group, preferably an amine group.

5. The method according to any one of the preceding claims, **characterized in that** the capping molecule is selected from the group consisting of cysteamine, 3-aminopropanethiol, 6-aminohexanethiol or trimethyl(10-sulfanyldecyl)azanium and mixtures thereof, preferably cysteamine.

6. The method according to any one of the preceding claims, **characterized in that** the pH value in step (d) is adjusted to pH 2 to 7, preferably to pH 3 to 6, most preferred to pH 3 to 5, and/or **in that** the non-ionic surfactant in step (e) is removed by centrifuging and washing or by dialysis, preferably by dialysis.

7. A surface-capped nanoparticle for adhesion to bacteria.

8. The surface-capped nanoparticle according to claim 7, **characterized in that** the nanoparticles are metallic nanoparticles, preferably noble metal, more preferably gold and/or silver, most preferred gold, and/or
the nanoparticles have an average diameter of from 5 nm to 500 nm, preferably from 10 nm to 300 nm, most preferred from 20 nm to 200 nm determined by transmission electron microscopy (TEM), and/or
the capping molecule is positively charged, preferably having the general formula (I) HS-(CR¹R²)ₙ-Y, wherein Y is a functional group with a positive charge or can be converted to have a positive charge by protonation, preferably an amine group or a quaternary ammonium group, most preferred an amine group, n is an integer from 2 to 15, preferably from 2 to 10, more preferably from 2 to 6, R¹ and R² are independently of each other H or C₁₋₃-alkyl, preferably H, and/or the capping molecule is selected from the group consisting of cysteamine, 3-aminopropanethiol, 6-aminohexanethiol or trimethyl(10-sulfanyldecyl)azanium and mixtures thereof, preferably cysteamine.

9. A surface-capped nanoparticle obtainable by the method according to any one of claims 1 to 6.

10. A method for detecting bacteria, the method comprising the steps of:
(f) taking a sample of a medium or a surface to be analyzed for the presence of bacteria;
(g) contacting the sample with the nanoparticles according to any one of claims 7 to 9 to form bacteria/nanoparticle complexes comprising bacteria and the nanoparticles according to any one of claims 7 to 9;
(h) optionally immobilizing the bacteria/nanoparticle complexes on an analysis surface;
(i) acquiring surface-enhanced Raman spectra (SERS) of the bacteria/nanoparticle complexes on the analysis surface;
(j) optionally identifying the bacteria applying machine learning techniques.

11. The method according to claim 10, **characterized in that** identifying the bacteria in step (e) comprises subjecting the SERS spectra to a Principal Component Analysis (PCA) algorithm.

12. The method according to any one of claims 10 or 11, **characterized in that** the method is suitable for detecting and/or identifying gram-positive bacteria and/or gram-negative bacteria, preferably belonging to the genus Pseudomonas, Escherichia, Salmonella and/or Listeria.

13. The method according to any one of claims 10 or 11, **characterized in that** the method is suitable for detecting and/or identifying gram-positive bacteria, preferably belonging to the genus Listeria.

14. The method according to any one of claims 10 to 13, **characterized in that** the method is suitable for detecting and/or identifying pathogenic bacteria.

15. The method according to any one of claims 10 to 14, **characterized in that** the method is suitable for detecting and identifying bacteria in 1 h or less, preferably in 30 min or less, and/or **in that** the method is suitable for detecting and identifying bacteria at concentrations in step (b) of as low as 10² CFU/mL.

16. The method according to any one of claims 10 to 15, **characterized in that** the method is suitable for detecting bacterial contamination in food and/or feed and/or pharmaceutical products and/or clinical settings, preferably in food and/or feed,
and/or **in that** the method is suitable for monitoring microbiological safety of production lines for food and/or feed and/or pharmaceutical products, preferably in food and/or feed.

17. Use of nanoparticles according to any one of claims 7 to 9 for detecting bacterial contamination in food and/or feed and/or pharmaceutical products and/or clinical settings, in particular by SERS,
or for monitoring microbiological safety of production lines for food and/or feed and/or pharmaceutical products, in particular by SERS.
